# EUROPEAN PATENT APPLICATION

(11) **EP 3 815 649 A1**
(43) Date of publication of application: **05.05.2021**
(21) Application number: 19205682.8
(22) Date of filing: 28.10.2019
(51) Int. Cl.: A61F 2/32, A61F 2/38, A61B 5/00, A61F 2/46, G16H 20/40

(54) **THERAGNOSTIC ENDOPROSTHETIC SPACER**

(71) Applicant: Klinikum rechts der Isar der Technischen Universität München, 81675 München (DE); Technische Universität München, 80333 München (DE)
(72) Inventor: Burgkart, Rainer, 80469 München (DE); Hayden, Oliver, 85368 Moosburg an der Isar (DE); Obermeier, Andreas, 80939 München (DE); von Eishenhart-Rothe, Rüdiger, 81929 München (DE)
(74) Representative: Lucke, Andreas

(57) **Abstract**

Disclosed herein is an endoprosthetic spacer (100, 200, 300) for administering a therapeutic treatment, in particular a theragnostic treatment. The endoprosthetic spacer (100, 200, 300) comprises a body (102) that is configured to replace at least a part of a bone, a sensor assembly (104) comprising at least one sensor (104A, 104B, 104C), a communication module (108) configured to transmit a signal; and a controller (106) configured to read out a sensor signal from the at least one sensor (104A, 104B, 104C) and to transmit an output signal (110) via the communication module (108).

## Description

### FIELD OF THE INVENTION

The present invention is in the field of medical technology. In particular, the invention relates to an endoprosthetic spacer for implantation in the human body.

### BACKGROUND

Endoprostheses are widely used for permanently replacing bones in the human body, in particular for replacing joints such as the hip joint, shoulder joint or knee joint. In recent years, sensor-equipped endoprostheses have been developed, which e.g. comprise a temperature or pH sensor for determining ambient conditions, see for example EP 3231360 A1. Sometimes, an infection may develop after implanting a prosthesis, which may require treatment and in some cases replacement of the prosthesis. For this, a two-stage procedure may be employed, wherein the infected prosthesis is removed and replaced by a temporary prosthesis or spacer. The spacer may remain in the patient for a couple of weeks until the infection is cured. Subsequently, the spacer may be removed and may be replaced by a permanent prosthesis again.

For treating the infection, the temporary spacer may contain an antibiotic that is continuously released from the spacer. CN 102415921 A discloses a spacer for replacing a broken bone which is equipped with channels and an inlet at which a fluid can be provided from the outside for delivering a drug via the channels to the surrounding tissue. To monitor the treatment, an arthroscopy may be performed or a sample may be extracted by arthrocentesis. These procedures, however, pose an additional risk of infection and involve complicated and time-consuming surgery and diagnostics. Patients often have to remain in the hospital for an extended amount of time. Furthermore, these methods only provide information at one point in time and no continuous monitoring is possible.

### SUMMARY OF THE INVENTION

The object of the invention is thus to provide an endoprosthetic spacer for the treatment of patients with infected prostheses that facilitates the treatment and monitoring thereof.

This object is met by an endoprosthetic spacer according to claim 1, a computer-readable medium according to claim 11 and a method of manufacturing an endoprosthetic spacer according to claim 13. Embodiments of the present invention are detailed in the dependent claims.

The endoprosthetic spacer according to the invention is for administering a therapeutic treatment, in particular a theragnostic treatment. The spacer comprises a body that is configured to replace at least a part of a bone. The spacer further comprises a sensor assembly comprising at least one sensor, a communication module configured to transmit a signal and a controller. The controller is configured to read out a sensor signal from the at least one sensor and to transmit an output signal via the communication module.

The endoprosthetic spacer is configured to be implanted in the human body, in particular to temporarily replace an endoprosthesis, e.g. an artificial bone or joint, or a part thereof. The endoprosthetic spacer or at least an outer surface thereof consists of or comprises a material suitable for implantation in the human body, e.g. a biocompatible metal alloy or a plastic, for example polymethylmethacrylate (PMMA). In some examples, the endoprosthetic spacer may be combined with another element or material to replace the endoprosthesis, e.g. a material like PMMA for adapting a shape of the endoprosthetic spacer to a patient and/or for attaching the endoprosthetic in the patient's body.

In the context of this application, administering a therapeutic treatment refers to any recurring process or action suitable to improve a medical condition of a patient over time. Accordingly, the implantation of a prosthesis or spacer in itself is not considered a therapeutic treatment. As detailed below, administering the therapeutic treatment may for example comprise releasing a pharmaceutical substance such as an antibiotic, antiseptic and/or any other drug, applying electromagnetic radiation such as light, in particular ultraviolet light, applying an electric current or a combination thereof. In some examples, the endoprosthetic spacer itself may be configured to administer the therapeutic treatment, for example as described below. In other examples, the endoprosthetic spacer may be configured to receive an element or device that is configured to administer the therapeutic treatment. The endoprosthetic spacer may for example be configured to be attached to a device that is configured to release a pharmaceutical substance or may be configured to be embedded in a material that is configured to release a pharmaceutical substance, e.g. PMMA mixed with the pharmaceutical substance.

In a preferred embodiment, the endoprosthetic spacer may be for administering a theragnostic treatment, wherein a theragnostic treatment refers to a combination of a therapeutic treatment and a diagnostic method. The diagnostic method may for example comprise performing a measurement with the at least one sensor and/or releasing a diagnostic substance from the endoprosthetic spacer, e.g. as detailed below.

The body of the endoprosthetic spacer is configured to replace a bone or a part thereof. For this, a shape and size of the body and/or mechanical properties of the body such as its stiffness may be adapted to the bone to be replaced, e.g. by choosing an appropriate material and/or dimensions for the body. The body may for example consist of or comprise a metal, a metal alloy, polyethylene, a ceramic, PMMA or a combination thereof. In some examples, the body may be a support structure that is to be combined with another element or material to replace the bone, e.g. to adapt the shape of the body to the respective bone.

The sensor assembly comprises at least one sensor, which may e.g. be attached to, connected to and/or housed within the body. The at least one sensor may for example be an optical sensor, an ambient sensor or a biomarker sensor as described in more detail below. In some examples, the sensor assembly may comprise a plurality of sensors, e.g. any combination of the aforementioned sensors. A sensor may be arranged such that it is enclosed by the body, i.e. such that the at least one sensor is not in contact with the environment of the endoprosthetic spacer. Alternatively, a sensor may be arranged such that at least a part thereof, e.g. a detection surface, is in contact with the environment of the endoprosthetic spacer.

The communication module is configured to transmit a signal, wherein the signal may be an analog signal or preferably a digital signal. The communication module may also be configured to receive a signal, e.g. a command signal as detailed below. The communication module may be implemented in hardware, software or a combination thereof. In some embodiments, the communication module may be integrated in the controller. The communication module may comprise an antenna configured to emit and/or receive an electromagnetic signal. In a preferred embodiment, the communication module is configured to communicate via a wireless communication standard, e.g. Bluetooth, Wi-Fi and/or near-field communication.

The controller is configured to read out a sensor signal from the at least one sensor and to transmit an output signal via the communication module. If the sensor assembly comprises more than one sensor, the controller may be configured to read out a sensor signal from each of the sensors. The controller may be implemented in hardware, software or a combination thereof. The controller may for example be a microcontroller comprising a processor and a storage medium storing instructions to be executed by the processor to provide the controller functionality described herein. In addition, the controller may comprise one or more input and/or output ports, e.g. to connect one or more sensors or the communication module to the controller.

The sensor signal may e.g. be an analog or digital signal characterizing a quantity measured by the sensor, for example a light intensity, a temperature or a biomarker concentration. The controller may be configured to process the sensor signal. In some examples, the controller may include an analog-digital converter to convert an analog sensor signal to a digital signal. The controller may in particular be configured to generate the output signal based on the sensor signal. The output signal may comprise the sensor signal or a part thereof or a processed sensor signal. The controller may also be configured to determine a control signal for administering the therapeutic treatment as detailed below. The controller may further be configured to process a command signal, e.g. a command signal received via the communication module as described below.

The endoprosthetic spacer according to the invention facilitates administering of a therapeutic treatment and monitoring the effect of the treatment. By integrating a sensor and a communication module into the endoprosthetic spacer, the treatment can be monitored continuously from the outside without requiring surgery. The sensor may e.g. measure an ambient parameter, detect a biomarker or record images or videos. Information regarding this measurement may be read out via the communication module. This may even allow for monitoring a patient remotely, e.g. by connecting the communication module to the internet. Thereby, the time that a patient has to be hospitalized as well as the risk of secondary infections may be reduced. Furthermore, the communication module and the controller may allow for better control of the therapeutic treatment as described below in more detail.

In a preferred embodiment, the body may comprise or consist of a transparent material, e.g. a material transmitting light in the visible, ultra-violet and/or infrared spectrum. The transparent material may in particular comprise or consist of a transparent or translucent biocompatible material such as a thermoplastic material, preferably polymethylmethacrylate (PMMA), which is also known as bone cement. In some embodiments, the body may additionally comprise other materials, in particular a metal, a metal alloy, polyethylene, ceramic or a combination thereof, e.g. as a support structure that is enclosed in the PMMA. In other examples, the entire body may consist of the transparent material. One or more sensors, e.g. an optical sensor, may be arranged in the transparent material. A sensor may for example be embedded in the transparent material, e.g. such that the sensor is completely surrounded by the transparent material. This may for example be advantageous for facilitating sterility of the endoprosthetic spacer, for protecting the sensor during implantation and for isolating the sensor from the environment of the endoprosthetic spacer. In some embodiments, the transparent material may be configured to alter light transmitted through the transparent material, e.g. to spectrally filter light, for example light emitted by a light source arranged in the transparent material and/or light detected by a sensor arranged in the transparent material.

The sensor assembly may for example comprise an optical sensor that is configured to measure a light intensity. Accordingly, the sensor signal may characterize the measured intensity. The optical sensor may e.g. comprise a photodiode or a CCD or CMOS sensor. The optical sensor may in particular comprise a camera, e.g. a camera having a CCD or CMOS sensor and imaging optics for imaging an object onto the sensor. In this example, the sensor signal and/or the output signal may be an image or a video stream. This may allow for visual inspection without requiring an arthroscopy. The sensor assembly may additionally comprise a light source, e.g. to illuminate the optical sensor and/or an object imaged onto the optical sensor. In some examples, the optical sensor may be configured to perform a spectroscopic measurement, e.g. by measuring a light intensity in a spectrally resolved way. For this, the optical sensor may be configured to adjust a wavelength of light emitted by the light source and/or detected by the optical sensor, e.g. by spectral filtering. In some examples, the optical sensor may be embedded in the transparent material, in particular such that it is completely surrounded by the transparent material.

Additionally or alternatively, the sensor assembly may comprise one or more ambient sensors that are configured to determine an ambient parameter, e.g. an ambient parameter of the environment of the endoprosthetic sensor. The ambient sensor may e.g. be arranged on or adjacent to an outer surface of the endoprosthetic sensor, e.g. an outer surface of the body. A detection surface of the ambient sensor may be in contact with the environment of the endoprosthetic sensor. The ambient sensor may for example be a temperature sensor that is configured to measure a temperature, e.g. a thermistor or thermocouple, or a pH sensor configured to determine a pH value, e.g. pH meter. Additionally or alternatively, the ambient sensor may be configured to determine an electrical signal, for example a voltage or current, and/or may be configured to determine an electrical parameter, e.g. an electrical impedance. In some examples, the ambient parameter may be a concentration of one or more substances, for example an ion concentration, an oxygen concentration or a carbon dioxide concentration.

Additionally or alternatively, the sensor assembly may comprise one or more biomarker sensors that are configured to detect a biomarker, in particular a biomarker associated with an immune response and/or an inflammation and/or substrates or parts of prokaryotic cells, e.g. bacteria. The biomarker sensor may for example be configured to determine a concentration of the biomarker, e.g. in the environment of the endoprosthetic spacer. The biomarker sensor may for example be arranged on or adjacent to an outer surface of the endoprosthetic spacer, e.g. an outer surface of the body. A detection surface of the biomarker sensor may be in contact with the environment of the endoprosthetic sensor. The biomarker sensor may for example be configured to detect C-reactive proteins, glucose, cathepsin, antimicrobial peptides, heat shock proteins, substrates or parts of prokaryotic cells such as bacteria, labelled microorganisms or a combination thereof.

The body of the endoprosthetic spacer may in particular be configured to replace a joint or a part thereof, e.g. by replacing the bone structure of the respective joint. Preferably, the endoprosthetic spacer is configured to be articulating, e.g. to mimic the functionality of the respective joint at least in part. The body may comprise an articulating member and a receiving member configured to movably receive the articulating member, e.g. such that an orientation of the articulating member can be changed relative to an orientation of the receiving member. The body may additionally comprise means for connecting or attaching the articulating member and the receiving member to each other, e.g. an artificial ligament or tendon.

In a preferred embodiment, the endoprosthetic spacer is configured to release one or more pharmaceutical substances, e.g. an antibiotic, antiseptic and/or any other drug. Release of the pharmaceutical substance constitutes an example of a therapeutic treatment. Additionally or alternatively, the endoprosthetic spacer may be configured to release one or more diagnostic substances, for example a dye, an antibody and/or a labelled substance, e.g. a fluorophore-labelled substance, for diagnostic monitoring. In some examples, the body may contain the diagnostic and/or pharmaceutical substance and may be configured to passively release the substance e.g. by diffusion or osmosis. In one example, the substance may be admixed to one or more components of the body, e.g. the transparent material, which may for example be a mixture of PMMA and an antibiotic. A concentration of the substance may be adapted to a desired release rate of the substance.

Additionally or alternatively, the endoprosthetic spacer may comprise means for actively releasing one or more pharmaceutical and/or diagnostic substances, e.g. based on a control signal generated by the controller. In this way, a therapeutic treatment or diagnostic substance may be administered automatically, without requiring any actions by the patient or a physician. Furthermore, the substance may be delivered directly to the affected part of the patient's body, thereby reducing exposure of other parts of the patient's body to the substance.

The endoprosthetic spacer may for example comprise a dispenser having a reservoir configured to store the pharmaceutical and/or diagnostic substance, e.g. dissolved in a fluid. The dispenser may be configured to release a predetermined amount of the substance and/or the fluid containing the substance from the reservoir. The dispenser may for example comprise a microfluidic structure that is in fluid communication with the reservoir and an environment of the endoprosthetic spacer, e.g. via an opening on an outer surface of the endoprosthetic spacer. The microfluidic structure may e.g. comprise one or more channels, micropumps, microvalves or a combination thereof. In some examples, the dispenser may comprise an exchangeable reservoir, e.g. a reservoir that can be removably coupled to the body of the endoprosthetic spacer.

The endoprosthetic spacer may further comprise a microfluidic circulation system that is configured to extract a fluid from the environment of the endoprosthetic spacer and to subsequently release the extracted fluid to the environment of the endoprosthetic spacer. The microfluidic circulation system may e.g. extract and release the fluid through an inlet and outlet, respectively, each of which may for example comprise one or more openings in an outer surface of the endoprosthetic spacer. For this, the microfluidic circulation system may e.g. comprise a pump. In one example, the microfluidic circulation system may be configured such that the inlet of the microfluidic circulation system is arranged suprapatellar, i.e. above the patella, and the outlet of the microfluidic circulation system can be arranged e.g. retropatellar or intercondylar, i.e. behind the patella or between the femoral condyles, when the endoprosthetic spacer is implanted in a knee joint.

The microfluidic circulation system may be in fluid communication with the dispenser, e.g. through a valve. In some examples, the dispenser and the microfluidic circulation system may be a joint microfluidic structure and may e.g. share elements such as a pump, channels and/or openings. The microfluidic circulation system may for example be used to circulate synovial fluid, e.g. to increase the homogeneity of the synovial fluid and/or rinse synovial cavities and/or to concentrate substances or particles contained in the synovial fluid via filtering of the circulated synovial fluid. This may increase the efficiency of a therapeutic treatment as well as the reliability of a diagnostic measurement.

In a preferred embodiment, the microfluidic circulation system also comprises a filter. The microfluidic system may be configured to pump the fluid through the filter, which may e.g. be arranged between the inlet and the outlet of the microfluidic circulation system. Preferably, the filter contains a pharmaceutical substance. The filter may for example be coated with an antibiotic, e.g. to kill bacteria absorbed by the filter. In some examples, the filter may be arranged adjacent to a sensor, e.g. in a field of view of an optical sensor such as a camera. This may for example allow for detecting objects or substances absorbed by the filter, e.g. bacteria or metabolic products, thereby facilitating the detection.

Preferably, the endoprosthetic spacer further comprises a light source configured to emit light, in particular ultraviolet (UV) light. The light source may for example be configured to emit monochromatic or broadband light with a wavelength between 200 nm and 380 nm. Additionally or alternatively, the light source may for example be configured to emit monochromatic or broadband light in the visible range and/or the near-infrared range. The light source may e.g. comprise a light-emitting diode (LED), in particular a UV LED. Light may also be used as a therapeutic treatment. UV light may e.g. be used for bacterial elimination due to its larger photon energies compared to visible light. The light source may be attached to, connected to and/or housed within the body. In some examples, the light source may be embedded in the transparent material, e.g. completely surrounded by the transparent material, and may be configured to emit the light through the transparent material. Alternatively, the light source may be arranged on an outer surface of the endoprosthetic spacer. The light source may be configured to adjust an intensity or power of the emitted light and/or an output duration, e.g. based on a control signal generated by the controller. In some examples, the light source may also be used for illumination in combination with an optical sensor. The light source may for example be used to directly illuminate a field of view of the optical sensor and/or to excite fluorescence in the field of view of the optical sensor. In one example, the endoprosthetic space may comprise or consist of multiple elements, e.g. a main body and an additional body, and the light source and the optical sensor may be arranged in different elements. The light source may e.g. be in an additional body emitting light towards the main body comprising the optical sensor. In some examples, the light source may be configured to emit light in two different wavelength ranges, e.g. UV light as a therapeutic treatment and visible light for diagnostic monitoring, e.g. to selectively excite a fluorophore.

In some embodiments, the controller is configured to control administering of the therapeutic treatment based on the sensor signal. The controller may for example determine a control signal for the dispenser and/or light source based on the sensor signal. The controller may e.g. determine an amount of a pharmaceutical substance to be released or an amount of light to be emitted, for example as detailed below. This may allow for automatically adjusting the treatment depending on its effect without human intervention, e.g. by implementing an appropriate feedback loop. Additionally or alternatively, the controller may e.g. determine an amount of a diagnostic substance to be released based on the sensor signal.

In a preferred embodiment, the controller is configured to receive a command signal via the communication module and to administer the therapeutic treatment based on the command signal, e.g. as described below. In this way, a physician may easily adjust the treatment, in some cases even remotely. In some examples, the controller may be configured to administer the therapeutic treatment based on a combination of the sensor signal and the command signal.

In addition, the endoprosthetic spacer may comprise other components, for example a power source such as a battery for providing a supply voltage for the controller, the sensor assembly and/or the communication module. In some examples, the endoprosthetic spacer may also comprise means for charging the battery, e.g. via inductive charging. The endoprosthetic spacer may also comprise a memory or data storage, e.g. to store data relating to the sensor signal.

The invention further provides a computer-readable medium storing instructions that, when executed by a processor, cause the processor to (1) read out a sensor signal from a sensor in an endoprosthetic spacer; (2) generate an output signal for transmission via a communication module of the endoprosthetic spacer, wherein the output signal is based on the sensor signal; and (3) determine a control signal for controlling administering of a therapeutic treatment, in particular a theragnostic treatment, by the endoprosthetic spacer. The numbering above is for clarity only and does not indicate a certain order of execution of the instructions. As far as technically feasible, the instructions may be executed in an arbitrary order and also simultaneously at least in part.

The computer-readable medium may for example be configured to be included in an endoprosthetic spacer as described above, e.g. to be connected to the controller or as part of the controller. A processor of the controller may be configured to execute the instructions stored on the computer-readable medium, e.g. to provide the functionality described in the following.

The instructions may cause the processor to read out the sensor signal from a sensor of the sensor assembly, e.g. an optical sensor, an ambient sensor or a biomarker sensor. The instructions may further cause the processor to control a measurement by the sensor, e.g. to initiate and terminate the measurement by sending corresponding trigger signals and/or to set parameters of the sensor and/or the measurement by sending a configuration signal. Parameters of the measurement may be specified by a measurement plan, which may for example be stored on the computer-readable medium or a memory of the controller. The measurements may be performed continuously, at one or more points in time or during one or more intervals in time, wherein the respective measurement periods may e.g. be specified by the measurement plan.

The instructions may further cause the processor to process the sensor signal, wherein the processing may e.g. comprise a selection of a subset of data points, an averaging of data points, a compression of the sensor signal or an extraction of measurement values characterized by the sensor signal. The instructions may further cause the processor to analyze the sensor signal, e.g. by comparing the sensor signal or a measurement value characterized by the sensor signal with a predetermined threshold or range.

The instructions may cause the processor to determine the output signal and to transmit the output signal via the communication module. The output signal is determined based on the sensor signal. The output signal may e.g. contain the sensor signal or a processed sensor signal, e.g. a time-averaged or compressed sensor signal or measurement values extracted from the sensor signal. In some examples, the output signal may be determined based on an analysis of the sensor signal. The output signal may e.g. indicate that a measurement value characterized by the sensor signal is above or below a predetermined threshold or is outside of a predetermined range. The instructions may cause the processor to transmit the output signal to a local computing device in direct communication with the communication module, e.g. via a wireless connection. Additionally or alternatively, the instructions may cause the processor to transmit the output signal to a remote computing device, e.g. via the internet.

The instructions further cause the processor to determine a control signal that controls administering of the therapeutic treatment. The control signal may for example characterize an amount of a diagnostic substance and/or an amount of a pharmaceutical substance to be released from the endoprosthetic spacer, e.g. from the reservoir of the dispenser. As used herein, the amount of a substance to be released may e.g. refer to a volume or weight of the substance or a fluid containing the substance that is to be released and/or a rate at which substance or fluid is to be released. Additionally or alternatively, the control signal may characterize an amount of light, e.g. ultraviolet light, to be emitted from the endoprosthetic spacer. As used herein, the amount of light to be emitted may e.g. refer to a power or intensity of the light to be emitted, a duration during which light is emitted and/or a repetition rate with which the emission of light is repeated. In some examples, the control signal may be a trigger signal, e.g. to switch the light source on or off or to open or close a valve. The instructions may further cause the processor to generate the control signal, e.g. to send the control signal to the dispenser and/or the light source. In some examples, the instructions may cause the processor to determine a plurality of control signals, e.g. one control signal characterizing an amount of a pharmaceutical substance to be released and one control signal characterizing an amount of a light to be emitted. In one example, the instruction may cause the processor to determine a control signal for a microfluidic circulation system, e.g. a microfluidic circulation system as described above, wherein the control signal may e.g. control an extraction rate of the microfluidic circulation system.

In a preferred embodiment, the control signal is determined based on the sensor signal, a command signal received via the communication module or a combination thereof. The control signal may for example be determined using the sensor signal as a feedback signal, e.g. by comparison of a measurement value with a reference value or reference range. For example, the amount of the pharmaceutical substance to be released and/or the amount of light to be emitted may be increased and/or decreased based on a deviation of the measurement value from the reference value or the reference range. Alternatively, the control signal may be determined using the command signal, which may e.g. be sent by a physician to adjust the therapeutic treatment. The command signal may for example be sent using a local computing device in direct communication with the communication module, e.g. via a wireless connection, or using a remote computing device, which may e.g. be in communication with the communication module via the internet. The command signal may for example contain a treatment plan specifying the amount of the pharmaceutical substance to be released and/or the amount of light to be emitted. In some examples, both the sensor signal and the command signal may be used for determining the command signal. The command signal may e.g. determine the reference value, the reference range or other feedback parameters such as a gain and/or may set a range within which the control signal may be adjusted based on the sensor signal.

The invention also provides a method of manufacturing an endoprosthetic spacer for administering a therapeutic treatment, in particular a theragnostic treatment. The method comprises (1) providing at least one sensor. The method further comprises (2) providing a controller. The controller is configured to read out a measurement signal from the at least one sensor and to transmit an output signal via a communication module. The method also comprises (3) forming a body of the endoprosthetic spacer comprising the at least one sensor and the controller. The body is configured to replace at least a part of a bone and to administer the therapeutic treatment. The numbering above is for clarity only and does not indicate a certain order of execution of the method. As far as technically feasible, the steps may be executed in an arbitrary order and also simultaneously at least in part.

The at least one sensor may for example be an optical sensor, an ambient sensor, a biomarker sensor or a combination thereof, e.g. as described above. The controller may for example be similar to the controller of the endoprosthetic spacer provided by the invention and described above. The body to be formed may also be similar to the body of the endoprosthetic spacer according to the invention.

Forming the body may comprise modifying a shape and/or size of the body or a source material that the body is to be formed of. The shape and/or size may e.g. be modified to adapt the body to a bone that is to be replaced or a prosthesis that is to be replaced. In some examples, the body may be formed manually, e.g. by a surgeon during surgery. Additionally or alternatively, the body may e.g. be formed by molding, milling and/or 3D printing. The body may for example be formed of a thermoplastic material, in particular polymethylmethacrylate (PMMA), metal, metal alloy, polyethylene, ceramic or a combination thereof. Forming the body may comprise providing a support structure, e.g. a support structure comprising or consisting of metal, metal alloy, polyethylene, ceramic or a combination thereof. Additionally, forming the body may comprise embedding the support structure in a modelling layer, e.g. a modelling layer formed of a thermoplastic material, in particular PMMA. In some examples, the body is configured to replace at least a part of a joint. Forming the body may comprise forming an articulating member of the body and a receiving member of the body that is configured to movably receive the articulating member.

Forming the body may also comprise attaching or connecting the sensor, the controller and/or the communication module to the body and/or embedding the sensor, the controller and/or the communication module in the body. Forming the body may further comprise connecting the sensor, the controller and/or the communication module to each other, e.g. via wires or cables. In some examples, the support structure may comprise the at least one sensor, the controller and/or the communication module.

Providing the at least one sensor may comprise providing an optical sensor, in particular an optical sensor comprising a camera and/or an optical sensor configured to perform a spectroscopic measurement. In some embodiments, the body or a part thereof may be formed from a transparent material, e.g. a transparent thermoplastic material such as PMMA. In one example, the entire body may be formed from a transparent material. Forming the body may comprise arranging a sensor, in particular an optical sensor, in the transparent material. The sensor may be embedded in the transparent material, e.g. such that the sensor is completely surrounded by the transparent material. Thereby, the sensor may be sealed off from an environment of the endoprosthetic sensor, e.g. a patient's body, without blocking light incident of the sensor.

Additionally or alternatively, providing the at least one sensor may also comprise providing an ambient sensor that is configured to determine an ambient parameter, in particular a temperature and/or a pH value, and/or a biomarker sensor that is configured to detect a biomarker, e.g. as described above.

In a preferred embodiment, forming the body comprises providing a pharmaceutical substance in the body, e.g. an antibiotic, an antiseptic and/or any other drug. Additionally or alternatively, forming the body may comprise providing a diagnostic substance in the body, for example a dye, an antibody and/or a labelled substance, e.g. a fluorophore-labelled substance, for diagnostic monitoring. The substance may for example be provided by admixing the substance or a powder or fluid containing the substance to the body or a part thereof. The substance may for example be added to a thermoplastic material, e.g. PMMA, that is used to form the body.

Additionally or alternatively, providing the pharmaceutical substance and/or the diagnostic substance may comprise providing a reservoir storing the substance, e.g. dissolved in a fluid. Providing the pharmaceutical substance and/or the diagnostic substance may also comprise providing a dispenser that is configured to release a predetermined amount of the substance from the reservoir. In some examples, the reservoir may be integrated into the dispenser. Forming the body may comprise attaching or embedding the reservoir and/or the dispenser to/in the body and/or connecting the dispenser to the controller, e.g. via a cable or wire.

The method may also comprise providing a light source that is configured to emit light, e.g. as described above. The light source may for example be an ultraviolet light source configured to emit ultraviolet light. In some examples, the light source may be provided as a separate unit and forming the body may for example comprise arranging the light source in the transparent material, e.g. such that the light source is completely surrounded by the transparent material. In other examples, the support structure may comprise the light source.

The method may further comprise providing and/or attaching additional elements such as a power source or a data storage. The method may also comprise providing instructions to be executed by a processor of the controller. The instructions may e.g. be provided on a computer-readable medium as described above. The method may further comprise adjusting initial settings of the controller. The settings may e.g. be a treatment plan determining an amount of the pharmaceutical substance to be released and/or an amount of light to be emitted. Additionally or alternatively, the settings may relate to a sensor and may e.g. be a measurement plan determining measurement parameters such as a duration and/or repetition rate of measurements. The method may further comprise providing a microfluidic circulation system, e.g. a microfluidic circulation system as described above.

### LIST OF FIGURES

In the following, a detailed description of the invention and exemplary embodiments thereof is given with reference to the figures. The figures show schematic illustrations of
Fig. 1: an endoprosthetic spacer for administering a therapeutic treatment according to an exemplary embodiment of the invention;
Fig. 2: an endoprosthetic spacer for administering a therapeutic treatment in accordance with another embodiment of the invention;
Fig. 3a: an endoprosthetic spacer having an articulating member and a receiving member according to an exemplary embodiment of the invention;
Fig. 3b: an endoprosthetic spacer with a microfluidic circulation system according to an exemplary embodiment of the invention;
Fig. 4: a block diagram representing a computer-readable medium in accordance with an embodiment of the invention; and
Fig. 5: a flow diagram illustrating a method of manufacturing an endoprosthetic spacer for administering a therapeutic treatment according to an exemplary embodiment of the invention.

### DESCRIPTION OF THE PREFERRED EMBODIMENTS

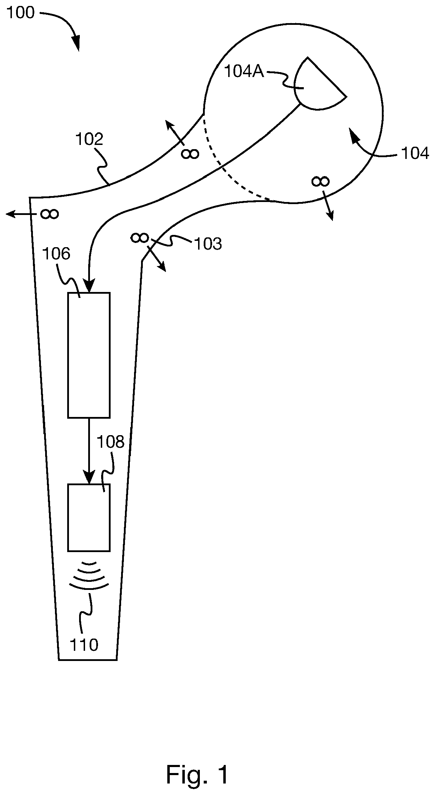

Fig. 1 depicts a schematic illustration (not to scale) of a sectional view of an endoprosthetic spacer 100 for administering a therapeutic treatment according to an exemplary embodiment of the invention. In this example, a body 102 of the endoprosthetic spacer 100 is configured to replace the head of the femur, i.e. the lower part of the hip joint. In other examples, the body 102 may additionally comprise a second member that is configured to replace the acetabulum, i.e. the upper part of the hip joint. The body 102 comprises or consists of a transparent material, preferably polymethylmethacrylate (PMMA). To administer the therapeutic treatment, a pharmaceutical substance 103, e.g. an antibiotic and/or antiseptic, is admixed to the transparent material such that the pharmaceutical substance 103 is continuously released from the body 102 to the surrounding environment. Thereby, the pharmaceutical substance 103 may be applied to an infected joint in a targeted fashion.

The endoprosthetic spacer 100 comprises a sensor assembly 104 with a sensor 104A. The sensor 104A is embedded in the body 102 such that the sensor 104A is completely surrounded by the transparent material. In other examples, the sensor 104A may be arranged such that it is in contact with an environment of endoprosthetic spacer 100. In the example of Fig. 1, the sensor 104A is an optical sensor 104A, in a particular a camera that is configured to image an interface between the endoprosthetic spacer 100 and the acetabulum. Preferably, the camera 104A also comprises a light source that is configured to illuminate the region to be imaged. In other examples, the sensor 104A may be a different type of optical sensor, for example a photodiode or an optical sensor configured to perform a spectroscopic measurement, or may be an ambient sensor or a biomarker sensor.

The camera 104A is connected to a controller 106 that is configured to read out a sensor signal from the camera 104A. The sensor signal for example contains an image or video recorded by the camera 104A. The controller 106 is configured to control the camera 104A, e.g. to trigger exposure of an image or to initiate and stop recording of a video.

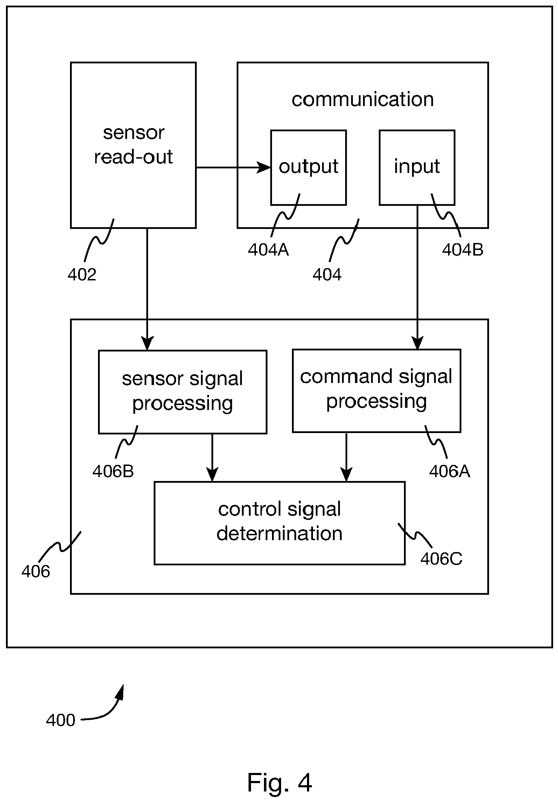

The controller 106 is a microcontroller that comprises a processor and a storage medium, e.g. a non-volatile memory like an EEPROM or flash memory. The storage medium stores instructions for execution by the processor to provide the functionality of the controller 106 described herein, in particular reading-out and processing the sensor signal. The storage medium may for example be the computer-readable medium 400 described below with reference to Fig. 4. The controller 106 additionally comprises a larger data storage or memory such as a flash memory for temporarily storing the sensor signal.

The controller 106 is connected to a communication module 108 that is configured to transmit a signal. In this example, the communication module 108 is a Bluetooth module that is configured to wirelessly communicate with other devices using the Bluetooth standard. The controller 106 is configured to transmit an output signal 110 via the communication module 108, wherein the output signal 110 may be used to transfer an image or video recorded by the camera 104A to an external computing device. The controller 106 is further configured to receive to an input signal via the communication module 108. The input signal may for example cause the controller 106 to record an image or video using the camera 104A or to initiate a data transfer of images or videos stored in the data storage. In this way, a physician may monitor the joint using the endoprosthetic spacer 100, e.g. to assess an effect of the therapeutic treatment.

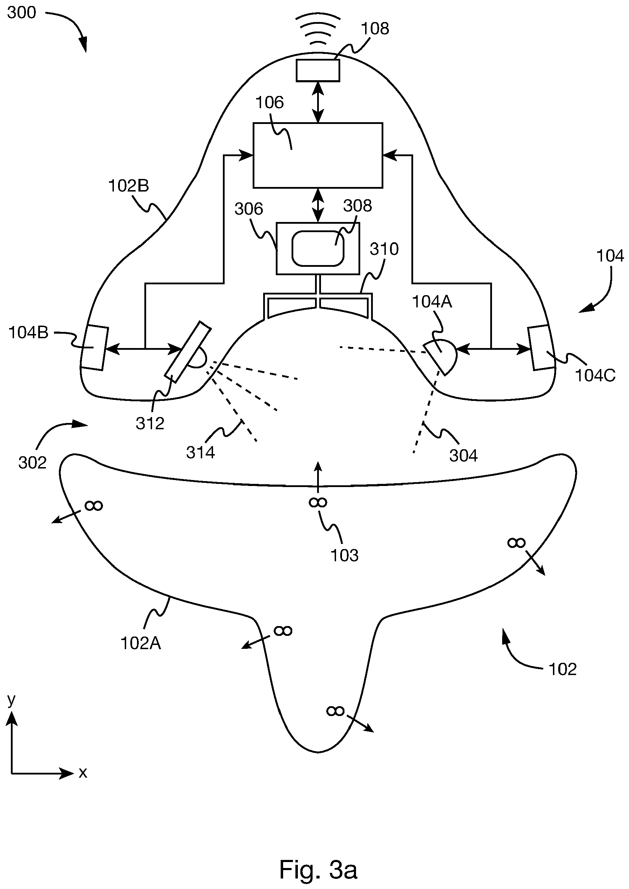

The endoprosthetic spacer 100 may also comprise a battery (not shown) to provide power for the camera 104A, the controller 106 and the communication module 108. The endoprosthetic spacer 100 may further comprise additional elements (not shown), e.g. additional sensors, a dispenser or a light source as detailed below with reference to Fig. 3a.

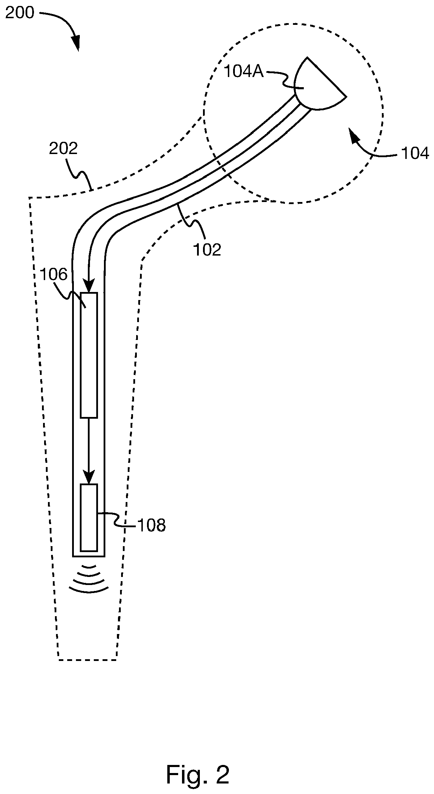

Fig. 2 depicts a schematic illustration (not to scale) of a sectional view of an endoprosthetic spacer 200 for administering a therapeutic treatment in accordance with another embodiment of the invention. Similar to the endoprosthetic spacer 100, the endoprosthetic spacer 200 comprises a body 102, a sensor assembly 104 with a sensor 104A, a controller 106 and a communication module 108. In contrast to the example of Fig. 1, the body 102 of the endoprosthetic spacer 200 is a support structure that may for example comprise a metal alloy such as a titanium alloy and/or polyethylene, in particular ultra-high molecular weight polyethylene (UHMW-PE). Accordingly, the shape and size of the body 102 resemble the bone to be replaced only approximately. The sensor 104A, the controller 106 and the communication module 108 are integrated in or attached to the body 102. The body 102 is configured to be embedded in a modeling layer 202, which may for example comprise or consist of a thermoplastic material like PMMA. In the example of Fig. 2, the body 102 is completely enclosed in the modeling layer 202. The shape and size of the modeling layer 202 closely resemble the bone to be replaced. The modeling layer 202 may for example be added before or during surgery, allowing for a flexible adjustment of the size and shape of the endoprosthetic spacer 200. The modeling layer 202 or the body 102 may comprise a pharmaceutical substance (not shown) similar to the example of Fig. 1. In other examples, the modeling layer 202 or the body 102 may additionally or alternatively comprise a diagnostic substance, e.g. a fluorophore-labelled antibody for diagnostic monitoring.

Fig. 3a shows a schematic illustration (not to scale) of a sectional view of an endoprosthetic spacer 300 with a body 102 having two members, an articulating member 102A and a receiving member 102B that is configured to movably receive the articulating member 102A. In the example of Fig. 3a, the endoprosthetic spacer 300 is configured to serve as a knee joint replacement, i.e. the articulating member 102A is configured to at least partially replace the distal end of the femur and the receiving member 102B is configured to at least partially replace the proximal end of the tibia. A distal surface of the receiving member 102B and a proximal surface of the articulating member 102A face each other across an interface volume 302 and are shaped such that the body 102 is configured to mimic the motional degrees of freedom of the human knee joint, e.g. such that the articulating member 102A may rotate with respect to the receiving member 102B around a rotation axis that is parallel to the horizontal x axis in Fig. 3a. In some examples, additional elements may be arranged in the interface volume 302, e.g. a joint liner or articulating surface (not shown). Similar to the endoprosthetic spacer 100, the body 102 is formed of a transparent thermoplastic material, preferably PMMA, and contains a pharmaceutical substance 103 that is admixed to the thermoplastic material. When implanted in a patient, the pharmaceutical substance 103 may slowly be released from the body 102 to the environment of the endoprosthetic spacer 300.

Similar to the endoprosthetic spacers 100 and 200, the endoprosthetic spacer 300 comprises a sensor assembly 104, a controller 106 and a communication module 108, each of which is embedded in the body 102, in particular in the receiving member 102B. In the example of Fig. 3a, the sensor assembly 104 comprises a plurality of sensors, namely a camera 104A, an ambient sensor 104B, and a biomarker sensor 104C.

The camera 104A comprises a camera sensor, e.g. a CMOS sensor (not shown) and imaging optics (not shown) configured to image a field of view 304 of the camera 104A onto the camera sensor. The camera 104A is fully enclosed by the thermoplastic material of the body 102 and is oriented such that the field of view 304 of the camera 104A faces the interface volume 302 between the articulating member 102A and the receiving member 102B. The camera 104A may additionally comprise a light source (not shown) that is configured to illuminate the field of view 304, for example one or more light emitting diodes. The camera 104A is connected with the controller 106, which is configured to control operation of the camera 104A and to read out a sensor signal from the camera 104A.

The ambient sensor 104B is a temperature sensor that is configured to measure a temperature at the position of the ambient sensor 104B. The ambient sensor 104B is arranged such that a detection surface of the ambient sensor 104B is in contact with the environment of the endoprosthetic spacer 300, e.g. to facilitate thermal equilibration. The ambient sensor 104B is connected with the controller 106, which is configured to read out a sensor signal from the ambient sensor 104B. The ambient sensor 104B may for example comprise a thermocouple and the controller 106 may be configured to measure a voltage across the thermocouple to determine the temperature. Alternatively, the ambient sensor 104B may for example comprise a thermistor and the controller 106 may be configured to measure a resistance of the thermistor to determine the temperature. In some examples, the ambient sensor 104C may comprise electronic circuits for generating a sensor signal that characterizes the measured temperature, e.g. a digital sensor signal.

The biomarker sensor 104C is configured to measure a concentration of a biomarker in the environment of the endoprosthetic spacer 300. Preferably, the biomarker is a biomarker associated with an immune response, for example an inflammation marker such as C-reactive protein (CRP). The biomarker sensor 104C is arranged in the body 102 such that a detection surface of the biomarker sensor 104C is in contact with the environment of the endoprosthetic spacer 300. The detection surface of the biomarker sensor 104C may for example comprise a plurality of field effect transistors (FETs) with CRP antibodies for immobilizing CRP in the vicinity of the field effect transistors (FETs) such that the presence of CRP can be inferred from a source-drain current of a FET. In other examples, different antibodies may be used for detecting other biomarkers. In particular, a plurality of different types of antibodies may be employed to simultaneously detect a plurality of biomarkers. The biomarker sensor 104C is also connected to the controller 106, which is configured to read out a sensor signal from the biomarker sensor 104C. The sensor signal may e.g. be an average source-drain current of the plurality of FETs. In other examples, the biomarker sensor 104C may comprise electronic circuits for generating a sensor signal that characterizes the biomarker concentration, e.g. a digital sensor signal.

In addition to and/or instead of the sensors shown in Fig. 3a, the sensor assembly 104 may comprise other sensors. The ambient sensor 104B may for example be a pH sensor that is configured to measure a pH value in the environment of the endoprosthetic spacer 300, e.g. via electrodes arranged on the detection surface, or may be a combined temperature and pH sensor that is configured to measure a temperature as well as a pH value. The sensor assembly 104 may also comprise an optical sensor that is configured to perform a spectroscopic measurement. The spectroscopic sensor may for example comprise a broadband or tunable light source, e.g. in the visible and/or near-infrared spectrum, and a detector configured to measure an intensity of light transmitted through or scattered and/or reflected by the environment of the endoprosthetic spacer 300, e.g. the interface volume 302 or surrounding tissue. The light source may be spectrally tunable and/or the detector may be configured to measure a spectrally resolved intensity. The controller 106 may be configured to determine spectra using the spectroscopic sensor, which may be transmitted via the communication module 108 and may for example be used to assess a tissue hydration or a hemoglobin concentration.

The endoprosthetic spacer 300 further comprises a dispenser 306 with a reservoir 308 that is configured to store a pharmaceutical substance, in particular a fluid containing the pharmaceutical substance (not shown). The pharmaceutical substance may be the same as the pharmaceutical substance 103 in the body 102 or may be different from the pharmaceutical substance 103. The volume of the reservoir 308 may for example be between 0.1 ml and 5 ml. The dispenser 306 is connected to the controller 106 and is configured to release a predetermined amount of the pharmaceutical substance from the reservoir to the environment of the endoprosthetic spacer 300, e.g. the interface volume 302. For this, the dispenser 306 comprises a microfluidic channel system 310 having a plurality of openings in fluid communication with the environment of the endoprosthetic spacer. The microfluidic channel system 310 may comprise a pump and/or a valve for releasing the pharmaceutical substance, e.g. in response to a control signal received from the controller 106. In other examples, the reservoir 308 may additionally or alternatively store a diagnostic substance, e.g. a dye or a fluorophore-labelled substance for diagnostic monitoring.

The endoprosthetic spacer 300 also comprises a light source 312 configured to emit light into an illumination cone 314, which in the example of Fig. 3a is oriented towards the interface volume 302. The light source is an ultraviolet (UV) light source configured to emit UV light, e.g. light between 200 nm and 380 nm. In other examples, the light source 312 may additionally or alternatively emit light in other wavelength ranges, for example in the visible range for illuminating the field of view 304 of the camera 104A or to excite a fluorophore. A maximum output power of the light emitted by the light source 312 may e.g. be between 0.1 mW and 10 mW. The light source 312 may be configured to adjust the output power, e.g. between 0 mW and the maximum output power. The light source 312 is connected to the controller 106 and is configured to receive a control signal from the controller 106, e.g. to switch the light source 312 on or off or to adjust the output power of the light source 312. The light source 312 may be a continuous-wave light source or may be a pulsed light source.

In other embodiments, the components of the endoprosthetic spacer 300 described above may be arranged differently. Some or all of the components may be arranged in the articulating member 102A. Components in different members of the body 102 may be connected via a cable between the members and/or a wireless communication link. The pharmaceutical substance 103 may be contained in the articulating member 102A and/or in the receiving member 102B. In some examples, the endoprosthetic spacer 300 may be similar to the endoprosthetic spacer 200 shown in Fig. 2, i.e. the body 102 may be a support structure that e.g. consists of or comprises a metal alloy and/or polyethylene and is configured to support a surrounding modelling layer, e.g. consisting of or comprising PMMA. The other components of the endoprosthetic spacer 300 may be embedded in or attached to the support structure as shown in Fig. 2. In some examples, the endoprosthetic spacer 300 may also be configured to replace a different joint or bone, e.g. the hip joint, wherein the receiving member 102B is shaped to at least partially replace the acetabulum and the articulating member 102A is shaped to at least partially replace the upper part of the femur.

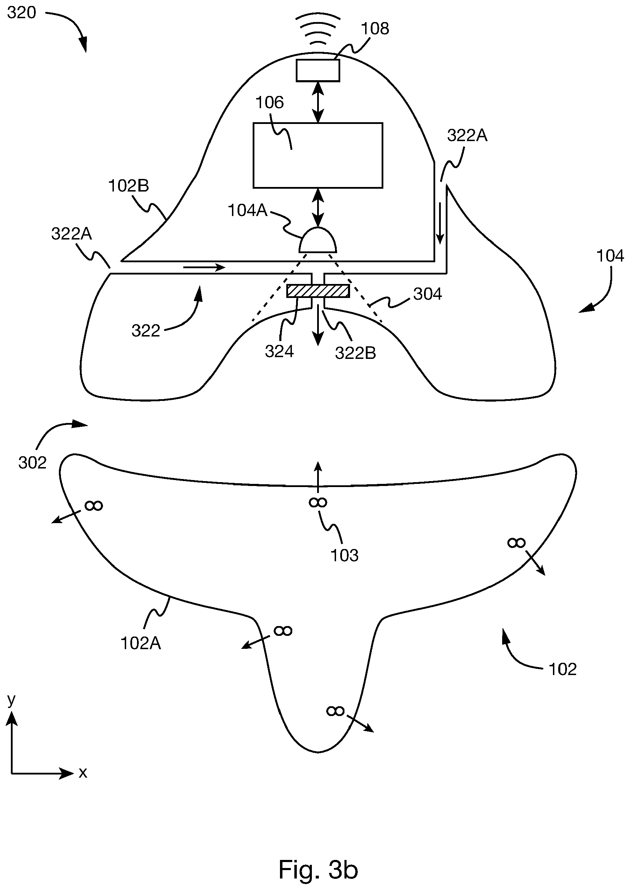

Fig. 3b depicts a schematic illustration (not to scale) of a sectional view of an endoprosthetic spacer 320. The endoprosthetic spacer 320 is similar to the endoprosthetic spacer 300 of Fig. 3a and also comprises a body 102 with an articulating member 102A and a receiving member 102B as well as a sensor assembly 104, a controller 106 and a communication module 108, each of which is embedded in the body 102.

The endoprosthetic spacer 320 comprises a microfluidic circulation system 322 with an inlet 322A formed by two openings, which may e.g. be arranged such that the openings are located suprapatellar when the endoprosthetic spacer 320 is implanted in a knee joint. The microfluidic circulation system 322 further comprises an outlet 322B, which may e.g. be arranged such that the opening is located retropatellar or intercondylar and faces the interface volume 302 when the endoprosthetic spacer 320 is implanted in a knee joint. The microfluidic circulation system 322 further comprises a pump (not shown) that is configured to pump fluid from the inlet 322 to the outlet as indicated by the arrows.

The microfluidic circulation system 322 additionally comprises a filter 324 that is arranged in a flow path between the inlet 322A and the outlet 322B. The filter 324 may e.g. be a bacterial filter that is configured to filter out bacteria and/or parts of bacteria from the fluid pumped by the microfluidic circulation system. The filter 324 may further be coated with an antibiotic and/or dye. In the example of Fig. 3b, the filter 324 is arranged in the field of view 304 of a camera 104A, which may e.g. be used to monitor material absorbed by the filter.

The endoprosthetic spacer 320 may comprises additional elements, e.g. as described above for the endoprosthetic spacer 300. In particular, the endoprosthetic spacer 320 may comprise a dispenser (not shown) with a reservoir and a microfluidic channel system. The microfluidic channel system may e.g. be coupled with the microfluidic circulation system through a microvalve. The dispenser and the microfluidic circulation system 322 may share a pump can be used for circulating fluid through the microfluidic channel system and for releasing fluid from the reservoir and/or may share one or more openings.

Fig. 4 schematically illustrates a computer-readable medium 400 in accordance with an embodiment of the invention. The computer-readable medium 400 comprises sets of machine-readable instructions for execution by a processor. Any of the sets of instructions referred to herein may be embodied by a corresponding software module. In other words, each of the blocks 402-406C may resemble a software module. The computer-readable medium 400 may for example be connected to or part of the controller 106 of the endoprosthetic spacer 100, 200 or 300, e.g. such that a processor of the controller 106 executes instructions stored on the computer-readable medium 400 to provide the functionality described in the following or at least a part thereof. Alternatively, the computer-readable medium 400 may for example be provided to copy instructions from the computer-readable medium 400 to an internal storage medium of one of the endoprosthetic spacers 100, 200 or 300. In the following, the computer-readable medium 400 is described with reference to Fig. 3a using the endoprosthetic spacer 300 as a non-limiting example.

The computer-readable medium 400 comprises a set of instructions 402 that, when executed by a processor, cause the processor to read out a sensor signal from a sensor in the endoprosthetic spacer 300, e.g. the camera 104A, the ambient sensor 104B and/or the biomarker sensor 104C. Accordingly, the set of instructions 402 may also be referred to as the sensor read-out instructions 402.

The sensor read-out instructions 402 additionally comprise instructions for performing a measurement, e.g. to send trigger signals to the camera 104A for initiating and terminating recording of a video, to the biomarker sensor 104C to initiate and terminate a biomarker concentration measurement or to a sub-module of the controller to initiate and terminate a voltage or resistance measurement of the temperature sensor 104B. The measurements are performed based on a measurement plan for the sensor assembly 104, which may e.g. be stored on the computer-readable medium 400 or the internal memory of the controller 106. The measurement plan specifies parameters of a measurement, e.g. how and when to perform a measurement. The measurement plan may for example define that an image is to be taken with the camera every 30 minutes or that a temperature measurement is to be performed every 30 seconds.

The sensor read-out instructions 402 also comprise instructions for processing the sensor signal, e.g. to average, filter and/or compress the sensor signal to reduce an amount of data to be transferred. The sensor read-out instructions 402 in particular comprise instructions for extracting measurement values characterized by the sensor signal from the sensor signal, e.g. converting a measured voltage or resistance from the temperature sensor 104B to a temperature using a predetermined calibration curve. Additionally, the sensor read-out instructions 402 also comprise instructions for analyzing the sensor signal, e.g. by comparing the sensor signal or a measurement value characterized by the sensor signal to a predetermined reference value or a predetermined reference range. The sensor read-out instructions 402 further comprise instructions for temporarily storing the sensor signal or the processed sensor signal on an internal memory of the controller 106, e.g. until an external computing device is coupled with the communication module 108.

The computer-readable medium 400 further comprises a set of instructions 404 that, when executed by a processor, provide capabilities for communication via the communication module 108, e.g. for communicating with an external computing device connected to the communication module 108 by a wireless communication link. Accordingly, the set of instructions 404 may also be referred to as the communication instructions 404. The communication instructions 404 comprise a subset of instructions 404A for transmitting signals via the communication module 108, also referred to as output instructions 404A, and a subset of instructions 404 B for receiving signals via the communication module 108, also referred to as input instructions 404B.

The output instructions 404A, when executed by a processor, cause the processor to generate an output signal for transmission via the communication module 108. The output instructions 404A are configured to generate different types of output signals. Some types of output signals are based on the sensor signal that is read out using the sensor read-out instructions 402. One type of output signal contains the sensor signal or a processed sensor signal. There may be different output signals for each of the sensors 104A-104C of the sensor assembly 104 or one output signal based on the sensors signals from all of the sensors 104A-104C. Another type of output signal is a warning signal, which indicates that a measurement value is below or above the predetermined reference value or outside of the predetermined reference range. Yet another type of output signal contains information regarding the status of the endoprosthetic spacer 300, e.g. a filling level of the reservoir 308, a treatment plan for the dispenser 306 and/or the light source 312, a measurement plan for the sensor assembly 104 and/or a charging status of a battery of the endoprosthetic spacer 300.

The input instructions 404B control the handling of input signals received by the communication module. The input instructions 404B are configured to handle different types of input signals. The input instructions 404B handle command signals for altering settings of the endoprosthetic spacer 300 or a component thereof or for controlling operation of a component of the endoprosthetic spacer 300. The command signal may e.g. contain a new measurement plan or a new treatment plan that is to replace a current measurement or treatment plan, respectively. Alternatively, the command signal may control one of the sensors 104A-104C, e.g. may trigger recording of a video by the camera 104C, or may directly control administering of the therapeutic treatment, e.g. by switching on the light source 312 or releasing pharmaceutical substance from the reservoir 308.

The computer-readable medium 400 further comprises a set of instructions 406 that, when executed by a processor, determine a control signal for controlling administering of a therapeutic treatment. The set of instructions 406 may also be referred to as the treatment instructions 406. The endoprosthetic spacer 300 is configured to administer two types of therapeutic treatments: application of the pharmaceutical substance via the dispenser 306 and application of UV light via the light source 312. In the following, the application of the pharmaceutical substance via the dispenser 306 is used as an example for illustration purposes. Application of UV light may be controlled by the treatment instructions 406 in a similar way.

The treatment instructions 406 comprise three subsets of instructions, a set of instructions 406A for processing a command signal, a set of instructions 406B for processing a sensor signal and a set of instructions 406C to determine the control signal based on the output of the two sets of instructions 406A and 406B. The control signal characterizes an amount of the pharmaceutical substance to be released from the reservoir 308. Subsequently, the controller 106 sends the control signal to the dispenser 306 to administer the therapeutic treatment. The control signal may for example be a trigger signal that opens a valve and/or switches on a pump in the microfluidic channel system 310.

The set of instructions 406A are configured to process a command signal. As described above, a command signal may contain information related to a treatment plan that determines administering of the therapeutic treatment and may be stored in the memory of the controller 106. The treatment plan defines parameters based on which the pharmaceutical substance is released by the dispenser 306, in particular a rate at which the pharmaceutical substance or the fluid containing the pharmaceutical substance is released. The treatment plan may for example specify a continuous flow rate from the reservoir 306 such as 1 µl to 50 µl per hour. Alternatively, the treatment plan may specify a predetermined fluid volume to be released from the reservoir and a repetition rate how often the predetermined fluid volume is to be released, e.g. 1 µl to 10 µl every hour. The treatment plan also contains parameters regarding the determination of the control signal based on the sensor signal as described below. A command signal may also directly control administering of the therapeutic treatment and may e.g. specify an amount of fluid that is to be released instantaneously as a one-off treatment.

The set of instructions 406B are configured to process a sensor signal or a processed sensor signal provided by the sensor read-out instructions 402. The instructions 406B implement a feedback loop that is configured to adjust administering of the therapeutic treatment based on the sensor signal. In particular, the instructions 406B adjust parameters of the treatment plan based on the sensors signal. As an example, the sensor signal may characterize a concentration of C-reactive protein (CRP) determined by the biomarker sensor 104C, which may serve as an indicator of inflammation. Accordingly, the instructions 406B increase or decrease a flow rate or predetermined fluid volume and repetition rate specified by the treatment plan depending on the concentration of CRP. The treatment plan may contain an upper and/or lower bound to specify a range within which the flow rate or predetermined fluid volume and repetition rate may be adjusted based on the sensor signal. In one example, the treatment plan may specify that the flow rate can be adjusted between 50% and 200% of a predetermined value.

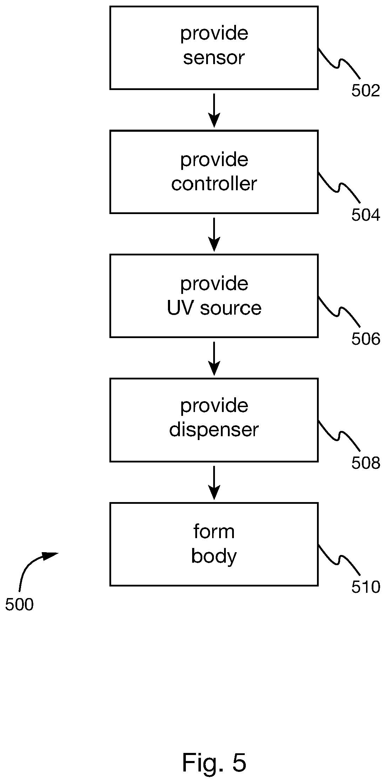

Fig. 5 shows a flowchart of a method 500 of manufacturing an endoprosthetic spacer in accordance with an embodiment of the invention. The method 500 may for example be executed during surgery to form a custom-built endoprosthetic spacer for subsequent implantation in a patient. Alternatively, the method 500 may be executed to manufacture pre-built endoprosthetic spacers, e.g. based on patient-specific designs or as a core structure to be adapted for a patient during surgery. In the following, the method 500 is described with reference to Figs. 2 and 3 using the endoprosthetic spacers 200 and 300 as non-limiting examples. The flowchart of Fig. 5 illustrates one example for an order of execution of the method 500. But the method 500 is not limited to this particular order of execution. As far as technically feasible, the steps of method 500 may be executed in an arbitrary order and also simultaneously at least in part.

In step 502, at least one sensor is provided. In the example of Fig. 3a, a camera 104A, an ambient sensor 104B and a biomarker sensor 104C are provided. The sensors may be provided as fully functional pre-built units or may be assembled during step 502, e.g. by combining a light source and a camera to build the light-source-equipped camera 104A.

In step 504, the controller 106 is provided that is configured to read out a measurement signal from the sensors 104A-104C and to transmit an output signal via the communication module 108. In some examples, step 504 also comprises programming the controller 106, e.g. by providing or employing the computer-readable medium 400. The instructions stored on the computer-readable medium 400 or a part thereof may for example be copied to a storage medium of the controller 106. In other examples, the controller 106 may already be provided including the computer-readable medium 400. Step 504 further comprises providing the communication module 108, either as part of the controller 106 or as an independent device. Step 504 may also comprise providing a measurement plan and a treatment plan, e.g. via the communication module 108 or on the computer-readable medium 400.

The method 500 also comprises, in step 506, providing the light source 312 and, in step 508, providing the dispenser 306. The light source 312 and/or the dispenser may be provided as fully functional pre-built units or may be assembled during step 502, e.g. by connecting the reservoir 308 to the microfluidic channel system 310. The dispenser 306 may be provided with an empty reservoir 308 or the reservoir 308 may be filled with a pharmaceutical substance or a mixture of pharmaceutical substances in step 508.

In step 510, the body 102 of the endoprosthetic spacer is formed. The body 102 of the endoprosthetic spacer 300 of Fig. 3a is for example formed from a thermoplastic material, preferably polymethylmethacrylate (PMMA), which is also referred to as bone cement. The bone cement is e.g. provided as a two-component system of a liquid containing methyl methacrylate (MMA) monomers and a powder containing pre-polymerized PMMA. The two components are mixed to initiate polymerization. One or more pharmaceutical substances 103 are provided by admixing the substances to the liquid and/or powder or adding the substances when mixing the two components. Subsequently, as the polymerization proceeds and the bone cement hardens, the articulating member 102A and the receiving member 102B are formed from the bone cement and adapted to the patient, e.g. based on the shape of a permanent prosthesis previously removed from the patient. The sensors 104A-104C of the sensor assembly 104, the controller 106, the communication module 108, the dispenser 306 and the light source 312 are connected to each other as described above with reference to Fig. 3a and, together with a power source, are embedded in the hardening bone cement, e.g. such that some or all of components are fully surrounded by bone cement and/or such that some or all of the components are arranged at an outer surface of the body 102. Subsequently, when the bone cement is completely or almost completely polymerized, the endoprosthetic spacer 300 may be implanted in the patient.

The body 102 of the endoprosthetic spacer 200 of Fig. 2, on the other hand, is for example formed from a metal alloy, polyethylene or a combination thereof. The body 102 may e.g. be formed by molding, milling, 3D printing or a combination thereof. The body 102 is shaped to roughly resemble the form of the bone to be replaced such that the shape of the endoprosthetic spacer 200 can be adapted to a specific patient later on, e.g. by surrounding the body 102 by the modelling layer 202. The body 102 may comprise cavities or recesses configured to receive components such as the sensor 104A, the controller 106 and the communication module 108 as well as additional components, e.g. a power source, a dispenser, a light source and/or other sensors. Alternatively, some or all of these components may be attached to outer surfaces of the body 102. The components may be glued to the body 102 or may be fastened using screws, clips or hooks. In some examples, step 510 may also comprise forming at least a part of the modeling layer 202, e.g. as described above using bone cement.

The embodiments of the present invention disclosed herein only constitute specific examples for illustration purposes. The present invention can be implemented in various ways and with many modifications without altering the underlying basic properties. Therefore, the present invention is only defined by the claims as stated below.

### LIST OF REFERENCE SIGNS

100 - endoprosthetic spacer
102 - body of the endoprosthetic spacer
102A - articulating member
102B - receiving member
103 - pharmaceutical substance
104 - sensor assembly
104A - optical sensor
104B - ambient sensor
104C - biomarker sensor
106 - controller
108 - communication module
200 - endoprosthetic spacer
202 - modelling layer
300 -endoprosthetic spacer
302 - interface volume
304 - camera field of view
306 - dispenser
308 - reservoir
310 - microfluidic channel system
312 - light source
314 - illumination cone
320 - endoprosthetic spacer
322 - microfluidic circulation system
322A - inlet of the microfluidic circulation system
322B - outlet of the microfluidic circulation system
324 - filter
400 - computer-readable medium
402 -sensor read-out instructions
404 - communication instructions
404A - output instructions
404B - input instructions
406 - treatment instructions
406A - instructions for processing of command signal
406B - instructions for processing of sensor signal
406C - instructions for determining the control signal
500 - method of manufacturing an endoprosthetic spacer
502 - step of providing a sensor
504 - step of providing a controller
506 - step of providing an ultra-violet light source
508 - step of providing a dispenser
510 - step of forming a body of the endoprosthetic spacer

## Claims

1. An endoprosthetic spacer (100, 200, 300) for administering a therapeutic treatment, in particular a theragnostic treatment, the endoprosthetic spacer (100, 200, 300) comprising:
a body (102) that is configured to replace at least a part of a bone;
a sensor assembly (104) comprising at least one sensor (104A, 104B, 104C);
a communication module (108) configured to transmit a signal; and
a controller (106) configured to read out a sensor signal from the at least one sensor (104A, 104B, 104C) and to transmit an output signal (110) via the communication module (108).

2. The endoprosthetic spacer (100, 200, 300) of claim 1, wherein the body (102) comprises a transparent material and the at least one sensor is an optical sensor (104A) arranged in the transparent material,
wherein the transparent material preferably comprises a thermoplastic material, in particular polymethylmethacrylate.

3. The endoprosthetic spacer (100, 200, 300) of claim 2, wherein the optical sensor (104A) comprises a camera and/or wherein the optical sensor (104A) is configured to perform a spectroscopic measurement.

4. The endoprosthetic spacer (300) of any one of the preceding claims, wherein the sensor assembly (104) comprises an ambient sensor (104B) that is configured to determine an ambient parameter, in particular a temperature and/or a pH value, and/or a biomarker sensor (104C) that is configured to detect a biomarker.

5. The endoprosthetic spacer (100, 200, 300) of any one of the preceding claims, wherein the body (102) is configured to replace at least a part of a joint,
wherein the body (102) preferably comprises an articulating member (102A) and a receiving member (102B) configured to movably receive the articulating member (102A).

6. The endoprosthetic spacer (100, 300) of any one of the preceding claims, wherein the endoprosthetic spacer (100) is configured to release a diagnostic substance and/or a pharmaceutical substance (103).

7. The endoprosthetic spacer (300) of claim 6, further comprising a dispenser (306) having a reservoir (308) configured to store the diagnostic substance and/or the pharmaceutical substance, wherein the dispenser (306) is configured to release a predetermined amount of the diagnostic substance and/or a predetermined amount of the pharmaceutical substance from the reservoir (308).

8. The endoprosthetic spacer (320) of any one of the preceding claims, further comprising a microfluidic circulation system (322) that is configured to extract a fluid from an environment of the endoprosthetic spacer (320) and to subsequently release the extracted fluid to the environment of the endoprosthetic spacer (320).

9. The endoprosthetic spacer (300) of any one of the preceding claims, further comprising a light source (312) configured to emit light, and/or
wherein the controller (106) is configured to control administering of the therapeutic treatment based on the sensor signal.

10. The endoprosthetic spacer (300) of any one of the preceding claims, wherein:
the communication module (108) is configured to receive a command signal; and
the controller (106) is configured to control administering of the therapeutic treatment based on the command signal.

11. A computer-readable medium (400) storing instructions that, when executed by a processor, cause the processor to:
read out a sensor signal from a sensor (104A, 104B, 104C) in an endoprosthetic spacer (300);
generate an output signal (110) for transmission via a communication module (108) of the endoprosthetic spacer (300), wherein the output signal (110) is based on the sensor signal; and
determine a control signal for controlling administering of a therapeutic treatment, in particular a theragnostic treatment, by the endoprosthetic spacer (300).

12. The computer-readable medium (400) of claim 11, wherein the control signal characterizes an amount of a diagnostic substance and/or an amount of a pharmaceutical substance to be released from the endoprosthetic spacer (300) and/or an amount of light to be emitted from the endoprosthetic spacer (300), and/or wherein the control signal is determined based on the sensor signal, a command signal received via the communication module (108) or a combination thereof.

13. A method (500) of manufacturing an endoprosthetic spacer (100, 200, 300) for administering a therapeutic treatment, in particular a theragnostic treatment, the method (500) comprising:
providing at least one sensor (104A, 104B, 104C);
providing a controller (106) that is configured to read out a sensor signal from the at least one sensor (104A, 104B, 104C) and to transmit an output signal (110) via a communication module (108); and
forming a body (102) of the endoprosthetic spacer (100) comprising the at least one sensor (104A, 104B, 104C) and the controller (106), wherein the body (102) is configured to replace at least a part of a bone and to administer the therapeutic treatment.

14. The method (500) of claim 13, wherein
providing the at least one sensor comprises providing an optical sensor (104A);
the body (102) comprises a transparent material, in particular polymethylmethacrylate; and
forming the body (102) comprises arranging the optical sensor (104A) in the transparent material, and/or
wherein providing the at least one sensor comprises providing an ambient sensor (104B) that is configured to determine an ambient parameter, in particular a temperature and/or a pH value, and/or a biomarker sensor (104C) that is configured to detect a biomarker.

15. The method (500) of any one of claims 13 and 14, wherein forming the body (102) comprises providing a diagnostic substance and/or a pharmaceutical substance (103) in the body (102),
wherein providing the diagnostic substance and/or the pharmaceutical substance preferably comprises providing a dispenser (306) with a reservoir (308) storing the diagnostic substance and/or the pharmaceutical substance, wherein the dispenser (306) is configured to release a predetermined amount of the diagnostic substance and/or a predetermined amount of the pharmaceutical substance from the reservoir (308), and/or
wherein the method (500) further comprises providing a light source (312) configured to emit light.
